# Europäisches Patentamt

# European Patent Office

# Office européen des brevets

(19)

(11) Numéro de publication: **0 205 721**
**B1**

(12) **FASCICULE DE BREVET EUROPÉEN**

(45) Date de publication du fascicule du brevet:
**02.08.89**

(51) Int. Cl.⁴: **C 02 F 3/28**, B 65 G 65/44

(21) Numéro de dépôt: **85870050.3**

(22) Date de dépôt: **02.04.85**

(54) **Cuve de fermentation anaérobie.**

(43) Date de publication de la demande:
**30.12.86 Bulletin 86/52**

(45) Mention de la délivrance du brevet:
**02.08.89 Bulletin 89/31**

(84) Etats contractants désignés:
**AT BE CH DE FR GB IT LI LU NL SE**

(56) Documents cité:
**FR-A-1 367 006**
**FR-A-2 257 505**
**FR-A-2 441 567**

(73) Titulaire: **Organic Waste Systems N.V., in het kort: O.W.S. N.V., Hansadok 403, B-2030 Antwerpen (BE)**

(72) Inventeur: **De Baere, Luc, Joos Lambrechtstraat 7, B-9000 Gand (BE)**

(74) Mandataire: **Bossard, Franz, 288, rue de Jamioulx, B-6110 Montigny- le- Tilleul (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 205 721 B1

## Description

Il est déjà connu de prévoir un dispositif d'extraction de matière poudreuse ou en forme de copeaux dans une cuve à fond plat de section circulaire ou polygonale. Le dispositif d'extraction connu se présente sous le forme d'un cadre coulissant sur le fond plat, associé à plusieurs couloirs ouverts vers le haut, parallèles au fond plat et équipés de vis de transport.

Le dispositif d'extraction connu agit de la manière suivante. Le cadre coulissant sur le fond plat râcle la matière jusqu'à ce qu'elle tombe dans un des couloirs ouverts vers le haut. Ensuite, les vis de transport déplacent la matière vers des ouvertures prévues sur le côté, en dessous du fond plat de la cuve.

L'invention a pour but d'adapter une telle cuve connue pour qu'elle puisse être utilisée comme cuve de réacteur à fermentation anaérobie.

Suivant l'invention, la cuve est caractérisée en ce qu'elle est fermée d'une part par un couvercle comprenant au moins une ouverture communiquant avec un conduit d'admission de matière à soumettre à une fermentation anaérobie et une ouverture communiquant avec un conduit d'évacuation de gaz produit lors de la fermentation, en ce que d'autre part les pistons ou tiges d'actionnement du cadre coulissant et les arbres des vis de transport sont entourés de joints hermétiques et en ce que finalement les couloirs équipés de vis de transport aboutissent à au moins un couloir d'évacuation dont la section se rétrécit du côté de l'ouverture de sortie et dans lequel est disposé une vis de transport dirigée vers l'ouverture de sortie, sans toutefois l'atteindre et qui engendre, dans la partie du couloir d'évacuation dont la section se rétrécit, un bouchon de matière fermentée de compacité suffisante pour empêcher l'échappement de gaz produit lors de la fermentation anaérobie par l'ouverture de sortie du couloir d'évacuation.

L'invention est expliquée ci-dessous en se référant à un exemple d'une forme d'exécution représenté schématiquement au dessin annexé. Les figures 1 et 2 du dessin sont respectivement une coupe d'une cuve en élévation et une coupe d'une cuve en plan. La figure 3 représente un dispositif de joints.

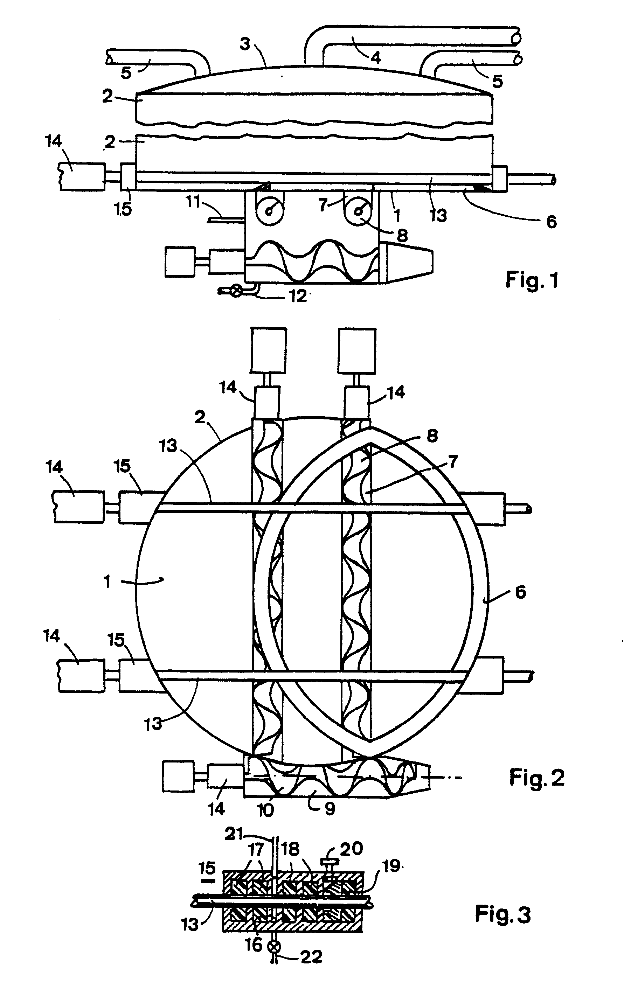

Aux figures 1 et 2 une cuve de fermentation est constituée d'un fond plat 1 d'une paroi latérale 2 et d'un couvercle 3. Dans le couvercle, une ouverture centrale communique avec un conduit de sortie 4 pour le gaz produit lors de la fermentation tandis que d'autres ouvertures, sont reliées à des conduits d'admission 5 de matière à soumettre à une fermentation anaérobie. Ces conduits d'admission sont alimentés par une ou plusieurs pompes du genre pompe à béton, non représentées. Grâce au fait de véhiculer au moyen de pompes la matière à soumettre à une fermentation, cette dernière atteint une compacité suffisante dans les conduits d'admission 5 pour empêcher l'échappement du gaz produit par les conduits d'admission 5. Si plusieurs conduits d'admission 5 aboutissent à des endroits différents sur le couvercle 3, il est possible de prévoir un agencement, non représenté, pour faire avancer à tour de rôle la matière dans les différents conduits d'admission 5 et assurer ainsi une bonne répartition de matière fraîche et éventuellement recyclée dans la partie supérieure de la cuve du réacteur. Dans le but d'évacuer la matière fermentée sur le fond de la cuve, un cadre coulissant 6 y exécute des mouvements de va et vient. Afin d'assurer un râclage certain, le cadre 6 est constitué de traverses en forme de coin orientées de manière à présenter la face du coin perpendiculaire au fond de la cuve du côté dirigé vers des couloirs 7 d'extraction. Ces couloirs d'extraction sont équipés de vis de transport 8 et communiquent avec un couloir d'évacuation 9 disposé sur un côté de la cuve.

La section d'au moins une partie du couloir d'évacuation 9 se rétrécit de plus en plus en s'approchant de l'ouverture de sortie de ce couloir 9. A l'intérieur du couloir 9 est disposé également une vis de transport 10 dirigée vers l'ouverture de sortie du couloir 9. L'extrémité aval de la vis 10 se trouve dans la partie du couloir 9 dont la section va en rétrécissant vers l'ouverture de sortie, mais n'atteint pas cette ouverture de sortie. De cette manière un bouchon de matière fermentée se forme en amont de l'ouverture de sortie.

Il est évidemment possible d'utiliser une vis de transport 10 dont le pas varie le long de l'axe et notamment dont le pas se raccourcit du côté aval c'est-à-dire à l'endroit où le couloir se rétrécit et où doit se former un bouchon d'une compacité suffisante pour éviter que le gaz de fermentation ne s'échappe par l'ouverture de sortie du couloir 9. L'espace au dessus de la vis 10, côté amont, c'est-à-dire du côté opposé à l'ouverture de sortie du couloir 9, peut être relié au moyen d'un conduit approprié 11 au conduit de sortie de gaz 4 partant du couvercle. De plus, le couloir d'évacuation 9 est de préférence incliné de sorte que la matière y est entraînée vers le haut et que le liquide essoré lors de la compression de la matière formant le bouchon coule vers le bas où peut être prévu un dispositif de purge 12, soit à action intermittente, soit à action permanente. L'extrémité de sortie du couloir 9 peut être fermé en outre par un régistre de fermeture, non représenté, pendant les phases d'arrêt de la vis de transport 10.

Le cadre 6 est fixé à une ou plusieurs tiges mobiles 13 actionnées par un ou plusieurs organes moteurs par exemple des vérins hydrauliques 14.

Aux endroits où ces tiges mobiles 13 traversent la cuve sont disposés des joints hermétiques 15 afin d'éviter que le gaz formé dans la cuve ne puisse s'échapper le long de ces tiges 13.

De tels joints 15 sont disposés aussi aux endroits où sortent les arbres des vis 8 ou 10.

A chaque endroit où sort une des tiges d'actionnement mobiles 13 de la cuve peut être prévu un dispositif à joints 15 selon la figure 3 comprenant un sas 16 entre deux joints 17 et 18. Un joint supplémentaire 19, inactif pendant le mouvement des tiges 13 et muni d'un dispositif de serrage 20 peut être prévu. Un tel joint 19 est appliqué sur la tige 13 grâce au dispositif 20 au moment d'un arrêt pour le remplacement des joints 17 et 18. La même disposition de joint peut être utilisée aux endroits où les arbres des vis de transport 8 ou 10 quittent l'enceinte enfermant le gaz de réaction, c'est-à-dire la cuve et les couloirs 7 et 9. Toutefois, comme les joints rotatifs autour des arbres des vis 8 et 10 subissent une usure relativement faible, il est possible de faire l'economie du joint 19 et de son dispositif de serrage 20.

Les sas 16 des divers dispositifs de joints 15 sont reliés au conduit de sortie de gaz 4 au moyen de conduits 21. De même, l'espace du couloir d'évacuation 9 en amont du bouchon à proximité de l'ouverture de sortie est relié au conduit 4 au moyen du conduit 11. De cette manière, on évite que des pressions excessives locales ne soient à la base de fuites vers l'extérieur de gaz de fermentation.

Comme le couloir 9, chaque sas 16 peut également être équipé d'un moyen de purge 22, soit permanent soit intermittent.

## Revendications

1. Cuve à fond plat comprenant un dispositif d'extraction composé d'un cadre coulissant (6) sur le fond plat, associé au moins à un couloir (7) ouvert vers le haut, parallèle au fond plat et équipé d'une vis de transport (8),

caractérisée en ce qu'elle est fermée d'une part par un couvercle (3) comprenant au moins une ouverture communiquant avec un conduit d'admission (5) de matière à soumettre à une fermentation anaérobie et une ouverture communiquant avec un conduit de sortie (4) de gaz produit lors de la fermentation, en ce que d'autre part au moins une tige (l3) d'actionnement du cadre coulissant (6) et les arbres des vis de transport (8 et 10) sont entourés de dispositifs de joints hermétiques et en ce que finalement, les couloirs (7) équipés de vis de transport (8) aboutissent à au moins un couloir d'évacuation (9) dont la section se rétrécit du côté de l'ouverture de sortie et dans lequel est disposé une vis de transport (10) dirigée vers l'ouverture de sortie, sans toutefois l'atteindre et qui engendre dans la partie du couloir d'évacuation (9) dont la section se rétrécit, un bouchon de matière fermentée de compacité suffisante pour empêcher l'échappement de gaz produit lors de la fermentation anaérobie par l'ouverture de sortie du couloir d'évacuation.

2. Cuve suivant la revendication 1, caractérisée en ce que les dispositifs de joint hermétiques comprennent un sas (16) entre deux joints et en ce que le ses (16) est relié au moyen d'un conduit (21) au conduit d'évacuation (4).

3. Cuve suivant la revendication 2, caractérisée en ce que les dispositifs de joint comprennent en outre un joint (19) disposé à la sortie de la cuve d'une tige d'actionnement (13) et muni d'un dispositif de serrage (20).

4. Cuve suivant une des revendications 1, 2 ou 3, caractérisée en ce que l'espace du couloir d'évacuation (9) en amont du bouchon obturant son ouverture de sortie est relié au moyen d'un conduit (11) au conduit d'évacuation (4).

5. Cuve suivant une des revendications précédentes, caractérisée en ce que le couloir d'évacuation (9) comprend un dispositif de purge (12).

6. Cuve suivant une des revendications 2 et 3, caractérisée en ce que chaque sas (16) est muni d'un moyen de purge (22).

## Patentansprüche

1. Behälter mit ebenem Boden und einer Fördervorrichtung bestehend aus einem auf dem ebenen Boden gleitenden Rahmen (6), zusammenarbeitend mit mindestens einem nach oben offenen Kanal (7), der parallel zum ebenen Boden läuft und mit einer Förderschnecke (8) ausgestattet ist,

dadurch gekennzeichnet, dass er einerseits durch einen Deckel (3) verschlossen ist und mindestens eine Öffnung besitzt, verbunden mit einem Zufuhrrohr (5) für das anaerobisch zu vergärende Gut, sowie eine Öffnung, verbunden mit einem Abfuhrrohr (4) für das durch die Gärung erzeugte Gas, dass andererseits wenigstens eine Betätigungsstange (13) des Gleitrahmens (6), sowie die Drehachsen der Förderschnecken (8, 10) durch hermetisch verschliessende Packvorrichtungen umgeben sind, und dass schliesslich die mit Förderschnecken (8) ausgestatteten Kanäle in mindestens ein Abförderrohr (9) münden, dessen Querschnitt sich gegen die Abförderöffnung hin verengert und in welchem eine Förderschnecke (10) sich in Richtung der Abförderöffnung ausdehnt, ohne diese jedoch zu erreichen und die im Teil des Abförderrohres (9) dessen Querschnitt sich verengert einen Stopfen aus vergorenem Gut erzeugt, dessen Kompaktheit genügend ist, um ein Ausdringen durch die Abförderöffnung des durch die anaerobe Gärung erzeugten Gases zu verhindern.

2. Behälter nach Patentanspruch 1, dadurch gekennzeichnet, dass die hermetischen Packvorrichtungen eine zwischen zwei Packungen angeordnete Schleusenkammer (16) aufweisen und dass die Schleusenkammer (16) über ein Rohr (21) mit dem Abfuhrrohr (4) verbunden ist.

3. Behälter nach Patentanspruch 2, dadurch gekennzeichnet, dass die Packvorrichtungen ausserdem eine Packung (19) aufweisen, die am Austritt aus dem Behälter einer Betätigungsstange (13) angeordnet ist und mit einer Blockierungsvorrichtung (20) versehen ist.

4. Behälter nach einem der Patentansprüche 1, 2 oder 3, dadurch gekennzeichnet, dass der Raum im Abförderrohr (9) vor dem Stopfen, der die Abförderöffnung verschliesst, durch ein Rohr (11) mit dem Abfuhrrohr (4) verbunden ist.

5. Behälter nach einem der vorhergehenden Patentansprüche, dadurch gekennzeichnet, dass das Abförderrohr (9) eine Ablassvorrichtung (12) aufweist.

6. Behälter nach einem der Patentansprüche 2 oder 3, dadurch gekennzeichnet, dass jede Schleusenkammer (16) eine Ablassvorrichtung (22) aufweist.

## Claims

1. Tank with a flat bottom comprising an extraction device consisting of a frame (6) sliding on the flat bottom and associated with at least one duct (7) which is open towards the top, parallel to the flat bottom and equipped with a conveyor screw (8), characterized in that it is closed, on the one hand, by a cover (3) comprising at least one opening communicating with a pipe (5) admitting material to be subjected to anaerobic fermentation and one opening communicating with a pipe (4) for discharging gas produced during fermentation, in that, on the other hand, at least one rod (13) for actuating the sliding frame (6) and the shafts of the conveyor screws (8 and 10) are surrounded by hermetic sealing devices and in that finally, the ducts (7) equipped with conveyor screws (8) lead to at least one discharge duct (9), the cross-section of which narrows in the region of the outlet opening and in which there is arranged a conveyor screw (10) which is directed towards the outlet opening, without however reaching it, and which produces, in the part of the discharge duct (9) whose cross-section narrows, a plug of fermented material sufficiently compact to prevent gas produced during anaerobic fermentation from escaping through the outlet opening of the discharge duct.

2. Tank according to Claim 1, characterized in that the hermetic sealing devices comprise a lock chamber (16) between two seals and in that the lock chamber (16) is connected by means of a pipe (21) to the discharge pipe (4).

3. Tank according to Claim 2, characterized in that the sealing devices comprise in addition a seal (19) arranged at the point where an actuating rod (13) leaves the tank and provided with a clamping device (20).

4. Tank according to one of Claims 1, 2 or 3, characterized in that the space inside the discharge duct (9) upstream of the plug closing its outlet opening is connected by means of a pipe (11) to the discharge pipe (4).

5. Tank according to one of the preceding claims, characterized in that the discharge duct (9) comprises a bleeding device (12).

6. Tank according to one of Claims 2 and 3, characterized in that each lock chamber (16) is provided with a bleeding means (22).

Fig. 1

Fig. 2

Fig. 3